(19) Europäisches Patentamt
European Patent Office
Office européen des brevets

(11) Publication number: **0 360 452 A2**

(12) # EUROPEAN PATENT APPLICATION

(21) Application number: 89308959.9

(51) Int. Cl.[5]: C08F 8/26 , G01N 33/53

(22) Date of filing: 05.09.89

(30) Priority: 06.09.88 US 240765

(43) Date of publication of application: 28.03.90 Bulletin 90/13

(84) Designated Contracting States: DE FR GB

(71) Applicant: EASTMAN KODAK COMPANY
343 State Street
Rochester New York 14650(US)

(72) Inventor: Warshawsky, Abraham c/o
EASTMAN KODAK COMPANY
Patent Department 343 State Street
Rochester New York 14650(US)
Inventor: Bale, Marsha Denise c/o EASTMAN KODAK COMPANY
Patent Department 343 State Street
Rochester New York 14650(US)

(74) Representative: Phillips, Margaret Dawn et al
Kodak Limited Patent Department Headstone Drive
Harrow, Middlesex HA1 4TY(GB)

(54) **Latex particles in analytical reagents, elements and methods.**

(57) There is disclosed a method of making a latex comprising an aqueous phase having dispersed therein non-porous monodisperse surfactant-free polymeric particles having benzaldehyde groups on the surfaces thereof wherein the polymer particles comprise from 5 to 100 weight percent of recurring benzaldehyde units. The method comprises the step of reacting a polymer having recurring vinylbenzylic halide units with an alkali nitroalkanenitronate and an alkoxide in the presence of water and a water-miscible organic solvent. The polymeric particles of the latex are useful in immunoassays.

EP 0 360 452 A2

## LATEX PARTICLES IN ANALYTICAL REAGENTS, ELEMENTS AND METHODS

The present invention relates to a method of making a polymeric latex; the polymeric latex; a water-insoluble reagent comprising the polymeric particles of the latex; and analytical elements and a method in which the reagent is used.

Immunoassays are widely used in clinical chemistry. Such assays are particularly advantageous in quantifying biological analytes which are present in very low concentration and cannot be adequately quantified by chemical techniques. Such analytes (called ligands herein) include therapeutic drugs, narcotics, antibiotics, hormones, proteins, and others known in the art. Several techniques have been devised for determining very low concentrations of ligands. For instance, a ligand may be labeled by various means to make it readily detectable. In competitive binding assays, a labeled ligand analog (identified as ligand analog herein) is placed in competition with unlabeled ligand for reaction with a fixed amount of the appropriate binding material (also called a receptor). Unknown concentrations of the ligand can be determined from the measured signal of either the bound or unbound ligand analog.

Biologically active polypeptides or proteins which are attached to insoluble polymeric particles through an aldehyde have been used in a variety of ways in immunoassays. For example, the diagnosis of pathological or other conditions in human beings and animals is often carried out using immunological principles for the detection of an immunologically reactive species (antibodies or an antigen) in the body fluids of mammals. Other proteins, such as enzymes, have been covalently linked to such particles for use in affinity chromatography, enzymatic reactions, specific binding reactions and immunoassays.

U.S. Patent 4,563,431 grafts methylamidoacetaldehyde onto polystyrene beads. The beads are designed for use in immunoassays. We have constructed beads of polystyrene having acrolein ($CH_2=CH-CH_2CHO$) polymerized onto the bead surface of polystyrene. The resulting beads contained a significant amount of aldehyde groups on the surface. However, the beads aggregated severely during immobilization of protein on the surfaces thereof. We believe the reason for this is that polyacrolein extends from the bead surface readily allowing crosslinking upon addition of the protein. Thus, one skilled in the art would expect the same with the beads of U.S. Patent 4,563,431. Also the patent teaches adding sodium dodecyl sulfate, a surfactant to the beads. Such surfactants interfere with coagulation in immunoassays. In addition, the synthetic process of the patent requires (1) blocking the aldehyde groups with acetal during polymerization to prevent reaction of the aldehyde groups with the monomers, and then (2) deblocking to recover the aldehyde groups.

Polymeric particles having aldehyde surface groups which are surfactant free, that do not aggregate during protein immobilization and which are easier to prepare then the particles disclosed in the above-cited prior art are needed for use in immunoassays.

The prior problems noted above are overcome with a latex composition characterized in that it comprises an aqueous continuous phase having dispersed therein non-porous monodisperse surfactant-free polymeric particles having aldehyde groups on the surfaces thereof; wherein the polymer particles comprise from 5 to 100 weight percent of recurring benzaldehyde units.

This latex composition makes possible a reagent in which the particles of the latex are covalently attached through the benzaldehyde groups to an immunological species which is capable of participating in an immunological reaction with a corresponding receptor.

The latex composition is made by reacting a polymer having recurring vinylbenzylic halide units with an alkali nitroalkanenitronate and an alkoxide in the presence of water and a water-miscible organic solvent. The latex composition cannot be made by prior art methods.

The present invention also provides an analytical element comprising:

(a) non-porous surfactant-free polymeric particles having aldehyde groups on the surface thereof and the polymeric particles comprising from 5 to 100 weight percent recurring benzaldehyde units;
and the particles are covalently attached through the aldehyde groups to

(b) an immunological species which is capable of participating in an immunological reaction with a corresponding receptor.

There is also provided:

(1) a method for the analysis of an immunological ligand in an aqueous fluid, said method comprising:

A. in the presence of an analog of said ligand, contacting a sample of said fluid with a reagent comprising:
(i) non-porous surfactant-free polymeric particles having aldehyde groups on the surface thereof and the polymeric particles comprise from 5 to 100 weight percent recurring benzaldehyde units;

and the particles are covalently attached through the aldehyde groups to

(ii) an immunological species which is capable of participating in an immunological reaction with said immunological ligand,

to form an immunological complex between said immunological species and both of said immunological ligand and ligand analog, and

B. determining the presence or amount of said immunological complex.

(2) an agglutination method for the analysis of a ligand in an aqueous liquid, which comprises:

A. contacting the liquid with a reagent comprising:

(i) non-porous surfactant-free polymeric particles having aldehyde groups on the surface thereof and the polymeric particles comprise from 5 to 100 weight percent recurring benzaldehyde units;

and the particles being covalently attached through the aldehyde groups to

(ii) an immunological species which is capable of participating in an immunological reaction with said ligand, so as to form an agglutinate of the reaction product of said ligand and said immunological species,

B. separating said agglutinate from unagglutinated materials, and

C. determining the presence or amount of said agglutinate.

The present invention provides highly sensitive reagents which can be used in a wide variety of immunological techniques. The benzaldehyde groups on the surfaces of the polymer particles readily react with proteins and other biological compounds which have free reactive amine groups. The reaction can be rapidly carried out under mild pH conditions and low temperatures. Agglutination, desensitization and instability that occur with known reagents are avoided. The conditions of attachment are not critical. Low temperatures, short times and flexible mixing conditions can be employed without sacrificing sensitivity.

The polymeric particles of the latex of the present invention and the reagents made therefrom can be used in many different immunoassays wherein the analyte (ligand) is an immunologically reactive species which has specific binding affinity for the immunological species of the reagent.

The reagent comprises an immunological species attached to the polymer particle. This species is a component of physiological fluids, cell and tissue extracts or a chemical compound that (a) has at least one reactive amine group and (b) is capable of participating in an immunological reaction with a corresponding receptor compound (natural or synthetic) which is a chemical or biological compound that has a reactive site for immunological reaction with the immunological species. By immunological species is meant either: (1) any substance which, when presented to an immunocompetent host, will result in the production of a specific antibody capable of binding with that substance, or (2) the antibody so produced, which species participates in an antigen-antibody reaction in the use thereof.

Representative immunological species include primary amines, amino acids, peptides, proteins, lipoproteins, glycoproteins, steroids, lipids, nucleic acids, hormones, vitamins, polysaccharides, glycolipids, alkaloids, organisms (bacteria, protozoa, fungi, viruses, rickettsia and the like) and components thereof, blood substances, tissue, biologically active materials conjugated with avidin or biotin and organ antigens and other materials known to one skilled in the art (see for example, U.S. Patent 4,181,636).

In some instances, the immunological species is an antibody which is directed against a hapten, drug (such as digoxin, phenytoin, phenobarbital, thyroxine, triiodothyronine, gentamicin, carbamazepine, primidone and theophylline), hormone, antibiotic or antigenic material, such as a protein, polypeptide, glycoprotein, polysaccharide, glycolipid and the like. Alternatively, the immunological species can be an antigen of some type (that is, a polypeptide or proteinaceous material) which is immunologically reactive with an antibody. In still other embodiments, the immunological species is an antibody which is directed against another antibody (that is, an anti-antibody). Both monoclonal and polyclonal antibodies can be used, and they can be whole molecules or various fragments thereof, as long as they have at least one reactive amine or sulfhydryl group which can be reacted with the pendant reactive groups of the polymeric particles.

In certain embodiments, the immunological species is an enzyme attached to the polymeric particle. Enzymes which can be attached in this manner include those which have reactive amine groups which can be reacted with the active groups on the polymer particles. Representative enzymes include aspartate aminotransaminase, alanine aminotransaminase, lactate dehydrogenase, creatine phosphokinase, gamma glutamyl transferase, alkaline acid phosphatase, and prostatic acid phosphatase. Methods of making such reagents are well known in the art.

The prior art reaction of polymers having pendant benzylic halide groups with sodium 2-nitropropanenitronate and sodium alkoxide, in anhydrous organic solvents [Fieser and Fieser, Organic Reagents, Vol. 1, page 1101, (1967); Organic Synthesis, Vol. 4, page 932 (1963) and Japanese Patent Reference 49/95930 (1974)] cannot be used to generate polymeric particles having surface aldehyde groups because uncrosslinked particles dissolve in the solvent. Crosslinked particles coagulate and cannot be redispersed. All the prior art methods of which we are aware teach the conversion of benzylic halide

groups to benzaldehyde groups in anhydrous solutions, sometimes at high temperatures above the melting point of the polymer particles.

Until the present invention it was impossible to make latex compositions of mono-disperse, surfactant-free polymer particles having benzaldehyde surface groups on the polymer beads.

We have found that a reaction employing any nitroalkanenitronate salt and an alkoxide salt can be employed in a medium comprising water and a miscible organic solvent to convert recurring benzylic halide units in particulate polymers to benzaldehyde units in the latex composition provided by the present invention without coagulation or dissolving. The polymer particles so prepared are clean and do not require further purification before use.

The method of making the latex of this invention involves the following reactions:

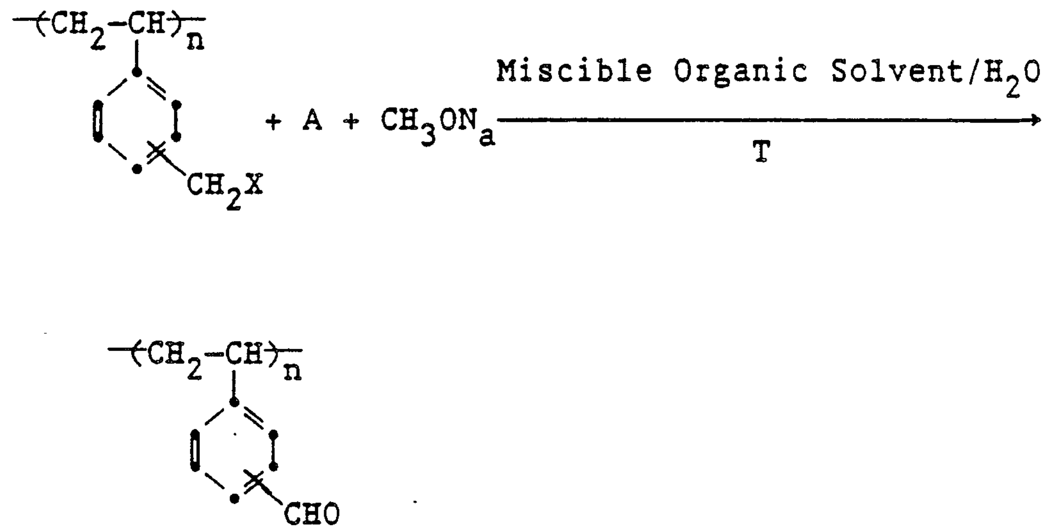


A represents 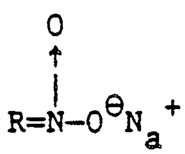

X represents Cl, Br
T represents 0-100 °C
R represents an alkylidene group of about 1 to 6 carbon atoms, such as isopropylidene.

The amount of water should be enough to prevent solvating the polymer particles by the organic solvent. The exact amount will depend on the particular organic solvent used. Generally about 5 percent to 95 percent, preferably less than 50 percent, will be sufficient. Almost any water-miscible organic solvent can be used. Examples include alcohols (ethanol, methanol, etc.), ethers (tetrahydrofuran and dioxane), 2-nitropropane, dimethylsulfoxide, etc.

The following example illustrates how the method is carried out.

Example 1 - Surface Modification of Poly(styrene-co-vinylbenzyl chloride) (Weight Ratio 70/30) Monodispersed 0.8µm Particles

A mixture of 250 ml of 0.5 M $NaOCH_3$, 12.5 ml of 2-nitropropane, and 50 ml of an aqueous suspension of 3.5% poly(styrene-co-vinylbenzyl chloride) particles was refluxed for two hours, then filtered by dropwise addition to the center of 230 Whatman Reeve Angel (RA 230) filter paper. In this example 2-nitropropane is at least partially converted to the 2-nitropropanenitronate salt. The particle surfaces showed chemical reactivity to dansylcadaverine which has an active amine group that reacts with aldehyde groups.

The water-insoluble reagent of the present invention is prepared by attaching the immunological species described above to a water-insoluble polymeric particle according to the invention.

The polymeric particles are water-insoluble latex particles having a particle size greater than about 0.01 micrometers, preferably in the range of from about 0.01 to about 3.0 micrometers.

The polymeric particles are composed of a polymer derived from at least one α,β-ethylenically unsaturated polymerizable monomer having pendant benzaldehyde groups. A number of representative monomers having the requisite pendant groups are known in the art.

Specifically useful polymers are those represented by the formula:

$-(A)_X-(B)_Y-(C)_Z-$

wherein A represents recurring units derived from one or more hydrophobic ethylenically unsaturated monomers,

B represents recurring units derived from an ethylenically unsaturated monomer having the formula:

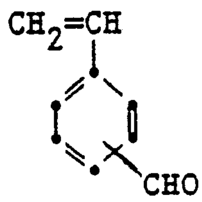 ;

C represents recurring units derived from one or more ethylenically unsaturated monomers other than those represented by A or B; and

x is from 0 to about 95 weight percent, y is from 5 to about 100 weight percent, and z is from 0 to about 20 weight percent.

Representative hydrophobic monomers of A include, but are not limited to, styrene and styrene derivatives (for example, vinyltoluene, 2,5-dimethylstyrene, 4-t-butylstyrene and 2-chlorostyrene), acrylic and methacrylic acid esters (for example, n-butyl acrylate, propyl methacrylate, methyl acrylate, ethyl methacrylate, 2-ethylhexyl methacrylate and methyl methacrylate) and vinyl acetate.

Particularly useful monomers from which A is derived are styrene, vinyltoluene, ethylene dimethacrylate, butyl acrylate, divinylbenzene, 2-ethylhexyl methacrylate and methyl methacrylate.

Generally C monomers have ionic or other hydrophilic groups which add dispersion stability to the resulting particles in aqueous solution. Useful ionic monomers include, but are not limited to, sodium 2-acrylamido-2-methylpropanesulfonate, sodium 3-acryloyloxypropanesulfonate, sodium acrylate, sodium methacrylate, and sodium styrenesulfonate, as well as other known sulfonates, sulfates, carboxylates, their salts or anhydrides, and useful nonionic polar monomers include 2-hydroxyethyl acrylate, 2,3-dihydroxypropyl acrylate, acrylamide, 2-hydroxyethyl methacrylate, N-isopropylacrylamide, 2-hydroxypropyl methacrylate, acrylonitrile and N-isobutoxymethyl acrylamide. Preferred monomers are sodium 2-acrylamido-2-methylpropanesulfonate, sodium acrylate, sodium 3-acryloyloxypropanesulfonate, sodium methacrylate, 2-hydroxyethyl acrylate, 2,3-dihydroxypropyl acrylate, acrylamide, N-isopropylacrylamide and acrylonitrile.

Representative polymers of which the particles are composed include poly(vinylbenzaldehyde), poly(vinylbenzaldehyde-co-vinylbenzyl chloride), poly(styrene-co-vinylbenzaldehyde), and poly(styrene-co-vinylbenzaldehyde-co-vinylbenzyl chloride).

The reagents of this invention are prepared by covalently attaching the immunological species to the particles as follows. The polymer particles are mixed with the immunological species in an aqueous buffered solution (pH generally from about 7 to about 10) and a concentration of from about 0.01 to about 40 weight percent polymer particles (preferably from about 0.01 to about 10 weight percent). The amount of immunological species is at a weight ratio of species to polymer of from about 0.1:1000 to about 1:10, and preferably from about 1:100 to about 1:10. Mixing is carried out at a temperature in the range of from about 5 to about 50 °C, and preferably from about 5 to about 25 °C, for from about 0.5 to about 48 hours. Any suitable buffer can be used, but a tertiary amine is preferred. The details of this procedure are illustrated in Example 2, infra.

The reagent of the present invention can be used in the qualitative or quantitative analysis of an analyte in aqueous liquids. Where the analyte is determinable by immunological methods, it is identified as a ligand herein. In particular, the invention can be used to assay biological fluids of animals, humans or plants, but preferably of humans. Such fluids include, but are not limited to, whole blood, plasma, sera, lymph, bile, urine, spinal fluid, sputum, perspiration and the like as well as stool secretions. It is also possible to assay fluid preparations of human or animal tissue such as skeletal muscle, heart, kidney, lungs, brains, bone marrow, skin and the like.

The reagent provided by the present invention can be used to detect and quantify any of a wide variety of ligands which are reactive with the immunological species on the reagent of the invention.

The reagent provided by the present invention can be used in a solution assay method in competitive binding immunoassays. By solution assay is meant that the reagents of this invention are used in liquid suspension in an immunoassay. Either bound (that is, complexed) or unbound (that is, uncomplexed) labeled materials can be determined. Physical separation of bound and unbound materials, if desired, can be carried out using any suitable separation technique. In using the analytical elements described below, either vertical or horizontal separation can be used.

In a competitive binding assay, the reagent provided by the present invention is generally present in a concentration in an amount which depends upon the amount of immunological species on the polymeric particles and the type of assay conducted. It also depends upon the binding constant of the immunological species. Suitable amounts for a given assay can be readily determined by one of ordinary skill in the art. The corresponding ligand analog can be present in amounts generally known in the art for a given assay. Other materials, for example, buffers, surfactants, reagents for color formation, can be used in the assay if desired.

An assay is generally carried out by physically contacting and mixing the reagent, the labeled ligand analog and the sample to be tested in a suitable container. The resulting suspension can be incubated, if desired, to promote complexation and other reactions. The sample is then evaluated using suitable detection equipment and procedures.

In another embodiment, the reagent provided by the present invention can be used in what are known in the art as immunometric assays, for example, "sandwich" assays. The details of such assays are provided in U.S. Patent 4,486,530. The reagent provided by the present invention is useful in such assays where the ligand to be determined has two or more epitopic sites for immunological reaction with two or more receptor molecules. The receptor molecules can be the same or different. One of the receptor molecules is identified herein as a first immunological species attached as a part of the reagent of this invention. A second immunological species is also used which is capable of immunologically reacting with the ligand at a site different than the site where the first species react. The result of the method is the formation of a ternary complex of the two distinct immunological species with the ligand. The second species (that is, the one not part of the reagent of this invention) is labeled in some manner so the resulting insoluble ternary complex can be readily detected. In a preferred immunometric assay, both immunological species are distinct antibodies directed against an antigen. They can be the same or different antibodies, whole or fragments, monoclonal or polyclonal.

In still another embodiment of this invention, the reagent provided by the present invention is comprised of an antigen and the ligand to be determined is an antibody. A second immunological species used in the assay is a second antibody which is directed against the first antibody. The second immunological species can be labeled or unlabeled. If the second antibody is unlabeled, a labeled third antibody directed against the second antibody, or a labeled second antigen molecule reactive with the second antibody can be used.

The methods employing the reagent provided by the present invention can also be practiced with a dry analytical element such as disclosed in U.S. Patent 4,670,381. The simplest element can be composed of an absorbent carrier material, for example, a thin sheet of a self-supporting absorbent or bibulous material, such as filter paper or strips, which has one or more zones, at least one zone containing the reagent of this invention. Other zones can be used to contain other useful reagents. Such elements are known in the art as test strips, diagnostic elements, dip sticks or diagnostic agents.

Useful absorbent carrier materials are insoluble and maintain their structural integrity when exposed to water or biological fluids such as whole blood or serum. Useful elements can be prepared from paper, porous particulate structures, porous polymeric films, cellulose, glass fibers, woven and non-woven fabrics (synthetic and non-synthetic) and the like. Useful materials and procedures for making such elements are well known in the art.

Preferably, the absorbent carrier material of the dry analytical element of this invention is a porous spreading zone. This zone can be self-supporting (that is, composed of a material rigid enough to maintain its integrity), but preferably it is carried on a separate support. Such a support can be any suitable dimensionally stable, and preferably, non-porous and transparent (that is, radiation transmissive) material which transmits electromagnetic radiation of a wavelength between about 200 and about 900 nm. A support of choice for a particular element should be compatible with the intended mode of detection (fluorescence, transmission or reflectance spectroscopy). Useful supports can be prepared from paper, metal foils, polystyrene, polyesters, polycarbonates or cellulose esters.

The porous spreading zone can be prepared from any suitable fibrous or non-fibrous material or mixtures of either or both. The void volume and average pore size of this zone can be varied depending

upon the use intended. Useful spreading zones can be prepared using materials and procedures described, for example, in U. S. Patents 4,292,272; 3,992,158; 4,258,001 and 4,430,436 and Japanese Patent Publication 57(1982)-101760.

The elements can have two or more discrete zones, either in the same layer or superimposed. At least one of the zones is preferably a porous spreading zone. The other zones can be reagent zones or registration zones as those zones are known in the art, additional spreading zones, radiation-blocking or filter zones, subbing zones or barrier zones. The zones are generally in fluid contact with each other, meaning that fluids, reagents and reaction products (for example, color dyes) can pass or be transported between superposed regions of adjacent zones. In other words, when the element is contacted with fluid, the reagents within the element become mixed and can readily interact. Preferably, each zone is a separately coated layer, although two or more zones can be separate areas in a single layer of the element. Besides the references noted above, suitable element components are described also, for example, in U. S. Patents 4,042,335; 4,132,528 and 4,144,306.

While the reagent provided by the present invention is located in the element, it is not critical in a competitive binding assay that the ligand analog also be located therein. It is possible to add the ligand analog to the element at the time of assay. Preferably, however, the ligand analog and the reagent of this invention are both in the element, and isolated from each other in such a manner such that they will not interact prior to the assay. For example, one or both of these materials can be encapsulated with a substance that will dissolve in the applied aqueous sample. Alternatively, they can be isolated by putting them in different zones of the element where they do not mix until the assay is carried out.

In one embodiment for a competitive binding assay, an analytical element for the immunological determination of a drug or hormone, comprises a non-porous support having thereon, in order and in fluid contact,

a reagent layer containing one or more reagents for providing a detectable signal in the determination,

a water-soluble layer containing an enzyme-labeled analog of the drug or hormone, and

a porous spreading layer containing a reagent provided by the present invention.

A variety of different elements, depending on the method of assay, can be prepared in accordance with the present invention. Elements can be configured in a variety of forms, including elongated tapes of any desired width, sheets, slides or chips.

The analytical methods can be manual or automated. In general, in using dry elements, analyte determination is made by taking the element from a supply roll, chip packet or other source and physically contacting it with a sample (for example, up to 500 µl) of the liquid to be tested so that the sample and reagents within the element become mixed. Such contact can be accomplished in any suitable manner, for example, by dipping or immersing the element into the sample or, preferably, by spotting the element by hand or machine with a drop of the sample with a suitable dispensing means. U.S. Patent 4,670,381, noted above, provides additional details of sample application. Wash fluids can also be used, as described, for example, in U.S. Patent 4,517,288.

After sample application, the element can be exposed to conditioning, such as incubation, heating or the like, that may be desirable to quicken or otherwise facilitate obtaining any test result.

In still another embodiment the reagent provided by this invention can be used in agglutination assays to determine the presence of a ligand which forms a complex with the immunological species in an immunological reaction. The resulting complex precipitates in a detectable agglutination or clumping of particles. The agglutination can be detected, for example, visually or with suitable light scattering detection equipment. Useful agglutination techniques are described in U.S. Patent 4,419,453.

The following examples illustrate the utility of the polymer particles of the latex of this invention in binding proteins and active antibodies.

Example 2 - Immobilization of Protein on Polymeric Particles Containing Benzaldehyde Groups

A sample of the polymeric particles having surface benzaldehyde groups prepared in Example 1 ($1 \times 10^{-3}$ g) and control particles from which the particles with surface aldehyde groups were prepared were incubated with 0.05 mg of bovine gamma globulin containing 0.1% $^{125}$I-labeled bovine gamma globulin (BGG) in 1 ml of buffer containing 0 or 10 mM of sodium cyanoborohydride (NaCNBH) to drive the covalent binding reaction.

The buffer, and time and temperature of incubation were chosen to be conducive to only reaction of primary amines of the protein with aldehyde (condition A) or to reaction with both aldehyde and benzylic halides (condition B). The conditions were as follows: condition A: 0.01 M tris(hydroxymethyl)aminomethane

(Tris) 0.15 M NaCl, pH 7.4; 4 hours; 22° C; condition B: 0.05 M sodium borate 0.15 M NaCl, pH 8.5; 24 hours; 37° C. Following the incubation, the particles and bound protein were separated from the unreacted protein by centrifugation.

Samples of the supernatant were analyzed for radioactivity and compared with that of the initial reaction mixture to determine the total amount of protein bound. The particles were washed once with reaction buffer, centrifuged, and resuspended in 1 ml of 1% sodium dodecyl sulfate in 0.2 M sodium phosphate buffer, pH 7.4. This surfactant removes any protein from the particle surface that is not covalently bound thereto. The particles were incubated for 24 hours at 22° C prior to centrifugation. The particles were washed 2 times with the surfactant solution to remove non-covalently bound protein and analyzed for radioactivity. This radioactivity was compared with the radioactivity of the initial mixture to determine the amount of protein covalently immobilized. The results are shown in Table I.

TABLE I

| Percent Bovine Gamma Globulin Covalently Bound to Particles | | | | | | | | |
|---|---|---|---|---|---|---|---|---|
| mM NaCNBH3 | Condition A | | | | Condition B | | | |
| | 0 | | 10 | | 0 | | 10 | |
| Particle: | Total | Covalent | Total | Covalent | Total | Covalent | Total | Covalent |
| Invention | 91 | 21 | 92 | 79 | 81 | 91 | 92 | 70 |
| Control | 49 | 3 | 44 | 23 | 37 | 39 | 34 | 18 |

These results demonstrate that the presence of active benzaldehyde on the surfaces of the particles are capable of excellent immobilization. Covalent binding on these particles is superior to that on the control particles under both conditions.

Example 3 -

Separate samples of particles having benzaldehyde surface groups of example 1 (30 mg) were incubated with 0.3 or 1.5 mg of a monoclonal antibody against phenobarbital (Phe 1.9) in 10 mL of buffer. The buffer was 0.01 M tris(hydroxymethyl)aminomethane (Tris), 0.15 M NaCl, pH 7.4. Incubation was for 2 hours at room temperature. At the end of this incubation, 1 mL of 30 mg/mL bovine serum albumin was added to cap unreacted bead surfaces thereby avoiding non-specific reactions with enzyme-antigen conjugates to be used subsequently in this example. Incubation was continued for 4 hours. The particles were transferred to a 50 mL centrifuge tube, diluted with phosphate buffered saline (PBS) and centrifuged. The supernatant was discarded and the particles resuspended in PBS. The particles were washed 2 more times in this fashion and resuspended finally in 4.6 mL PBS with 0.2% $NaN_3$ and stored at 4° C until use.

An identical experiment to that described above using $^3$H-bovine gamma globulin instead of Phe 1.9 was performed to determine the mass of protein bound to the bead surface under these conditions. In this case, a 0.5 mL aliquot of the final reaction mixture was monitored for radioactivity. One mL aliquots were taken and sedimented; a 0.5 mL sample of the supernatant was obtained and monitored for radioactivity. The pellet was washed with PBS and resuspended in 0.5 mL PBS and monitored for radioactivity. To the remaining 8 mL of the reaction mixture, 0.8 mL of 10% sodium dodecyl sulfate was added, and the mixture incubated at 37° C for 24 hours to strip non-covalently bound protein from the surface. Samples of the reaction mixture, supernatant, and washed pellet were obtained and monitored for radioactivity as described above. The amount of bovine gamma globulin bound to the particles (total and through covalent bonds) was calculated. We assumed the total and covalently bound Phe 1.9 on the particle surfaces to be the same.

Particles containing Phe 1.9 were diluted with PBS to give 4.0 x $10^{-7}$ M antibody binding sites based on the total mass of $^3$H-bovine gamma globulin bound to the bead surface and assuming each bound antibody has two binding sites. Dilutions were made of this solution to give a range of theoretical antibody binding sites from 0.063 to 100 nM. One hundred µL samples of diluted beads were mixed with 100 µL glucose oxidase-phenobarbital conjugate at 1 x $10^{-9}$ M in PBS with 1% BSA (Bovine Serum Albumin). After 3.5 hours, the particles were centrifuged, and 100 µL of the supernatant was transferred to a microtiter

plate. The supernatant was assayed for glucose oxidase activity. The results were determined as the amount of glucose oxidase-phenobarbital activity remaining following reaction with the antibody bearing particles as a function of antibody particles in the reaction. The concentration of theoretical antibody binding sites required to remove 50% of the initial enzyme-drug conjugate is used as a measure of the activity of the bound antibody. A low number of theoretical binding sites for 50% binding indicates greater activity of the immobilized antibody.

The results show that Phe 1.9 immobilized on the particles exhibit more than sufficient activity for use in immunoassays. Only 6.0 to 11.0 nM of binding sites were required to bind 50% of the glucose oxidase-phenobarbital conjugate. Further, Phe 1.9 immobilized on the particles gives similar activity whether immobilization occurs at low or high protein concentration, whereas the activity of Phe 1.9 on the parent beads is greater when immobilized at high protein concentration.

## Claims

1. A method of making a latex comprising an aqueous phase having dispersed therein non-porous monodisperse surfactant-free polymeric particles having aldehyde groups on the surfaces thereof; wherein the polymer particles comprise from 5 to 100 weight percent of recurring benzaldehyde units, characterized in that a polymer having recurring vinylbenzylic halide units with an alkali nitroalkanenitronate is reacted with an alkoxide in the presence of water and a water-miscible organic solvent.

2. A latex composition characterized in that it comprises an aqueous continuous phase having dispersed therein non-porous monodisperse surfactant-free polymeric particles having aldehyde groups on the surfaces thereof; wherein the polymer particles comprise from 5 to 100 weight percent of recurring benzaldehyde units.

3. The composition of claim 2 wherein the polymer of the particles is represented by the formula:

$-(A)_x-(B)_y-(C)_z-$

wherein A represents recurring units derived from one or more hydrophobic ethylenically unsaturated monomers,

B represents recurring units derived from an ethylenically unsaturated monomer having the formula:

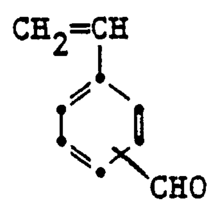

;

C represents recurring units derived from one or more ethylenically unsaturated monomers other than those represented by A or B; and

x is from 0 to about 95 weight percent, y is from 5 to about 100 weight percent, and z is from 0 to about 20 weight percent.

4. The composition of claim 3 wherein the polymer particles range in size from 0.1 to 3.0 µm.

5. The composition of claim 3 wherein the polymer is selected from the group consisting of poly(vinylbenzaldehyde), poly(vinylbenzaldehyde-co-vinylbenzyl chloride), poly(styrene-co-vinylbenzaldehyde), and poly(styrene-co-vinylbenzaldehyde-co-vinylbenzyl chloride).

6. A reagent comprising:

(a) non-porous surfactant-free polymeric particles having benzaldehyde groups on the surface thereof and the polymeric particles comprising from 5 to 100 weight percent recurring benzaldehyde units; and the particles are covalently attached through the aldehyde groups to

(b) an immunological species which is capable of participating in an immunological reaction with a corresponding receptor.

7. The reagent of claim 6 wherein said immunological species is an enzyme, drug, antibiotic or antibody.

8. The reagent of claim 6 wherein the polymer has the formula:

$-(A)_x-(B)_y-(C)_z-$

wherein A represents recurring units derived from one or more hydrophobic ethylenically unsaturated monomers,

B represents recurring units derived from an ethylenically unsaturated monomer having the formula:

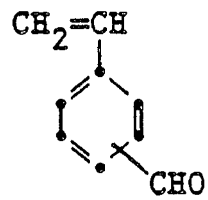 ;

C represents recurring units derived from one or more ethylenically unsaturated monomers other than those represented by A or B; and

x is from 0 to about 95 weight percent, y is from about 5 to 100 weight percent, and z is from 0 to about 20 weight percent.

9. The reagent of claim 6 wherein the polymer is poly(styrene-co-vinylbenzaldehyde-co-vinylbenzyl chloride).

10. The reagent of claim 9 wherein the polymer particle size is from 0.1 to 3.0 µm.

11. The reagent of claim 6 wherein the antibody is selected from antibodies directed against digoxin, phenytoin, phenobarbital, thyroxine, triiodothyronine, gentamicin, carbamazepine, primidone, tobramycin or theophylline.

12. An analytical element comprising an absorbent carrier material having one or more zones, and containing in one or more of said zones a reagent according to claims 6, 7, 8, 9, 10 and 11.